# EUROPEAN PATENT SPECIFICATION

(11) **EP 3 654 857 B1**
(45) Date of publication and mention of the grant of the patent: **08.07.2026**
(21) Application number: 18755903.4
(22) Date of filing: 18.07.2018
(51) Int. Cl.: A61B 17/221

(54) **UNIVERSAL RETRIEVAL DEVICE FOR REMOVING OBSTRUCTIONS FROM BODY LUMENS**
UNIVERSELLE BERGUNGSVORRICHTUNG ZUR ENTFERNUNG VON OBSTRUKTIONEN AUS KÖRPERLUMEN
DISPOSITIF DE RÉCUPÉRATION UNIVERSEL POUR ÉLIMINER DES OBSTRUCTIONS DE LUMIÈRES CORPORELLES

(30) Priority: 19.07.2017 US 201715653737
(43) Date of publication of application: 27.05.2020
(73) Proprietor: Hoya Corporation, Tokyo 160-8347 (JP)
(72) Inventor: KHANICHEH, Azadeh, Somerville, Massachusetts 02144 (US); OSTROVSKY, Isaac, Wellesley, Massachusetts 02481 (US); VELAGIC, Almir, Watertown, Massachusetts 02472 (US); POORE, Matthew Layton, Charlotte, North Carolina 28277 (US)
(74) Representative: Dennemeyer & Associates S.A.
(86) International application number: PCT/IB2018/055353
(87) International publication number: WO 2019/016736

(56) References cited:
- DE-A1- 3 407 708
- US-A- 5 944 728
- US-A1- 2013 023 894
- US-A1- 2013 131 688
- US-A1- 2016 151 080

## Description

### BACKGROUND

### Related Applications

This application claims the benefit of priority of U.S. patent application no. 15/653,737, filed July 19, 2017.

### Technical Field

The present disclosure generally relates to endoscopic devices and methods of use. More particularly, and without limitation, the disclosed embodiments relate to devices and methods for removing undesirable objects, such as obstructions, from body lumens. The methods disclosed herein, however, do not form part of the claimed invention.

### Background Description

The presence of objects in the lumen of a patient, such as kidney stones, biliary stones, gallstones, and the like may pose a health risk. Accordingly, removal of such objects is, when indicated, desirable. Tools have been devised to assist surgeons to accomplish this removal. In the case of biliary or pancreatic ductal systems, there are, generally speaking, two classes of tools available for removal of objects during an Endoscopic Retrograde Cholangio-Pancreatography (ERCP) procedure: retrieval balloons and retrieval baskets.

An exemplary catheter-mounted retrieval balloon may be mounted on an endoscope. The retrieval balloon is inserted into a patient while deflated, and navigated within the lumen to a position past the object to be removed. Once the balloon has been properly positioned in the lumen with respect to the object, the clinician will inflate the balloon. The inflation and expansion of the balloon presses allowing the target objects, *e.g.*, bile stones, to be swept out or the lumen along with the balloon as the catheter is withdrawn. Generally, balloons are effective for smaller stones of a diameter less than about 1 cm.

In situations where the target object is relatively large or of a shape rendering it difficult for balloon retrieval, a basket may be employed. A basket is essentially an expandable wire enclosure connected to a shaft configured for catheter delivery. In a delivery state, the basket resides in a collapsed configuration within the lumen of the delivery catheter. The end of the catheter is positioned proximate to the target object, and the shaft carrying the basket is urged distally with respect to the catheter end. The collapsed basket expands upon emerging from the catheter. Once expanded, the clinician maneuvers the device in an effort to capture the target object inside of the basket. Upon such entrapment, the shaft is withdrawn proximally with respect to the catheter, drawing the basket into the lumen and collapsing the basket's wire frame around the target object. Once collapsed, the catheter is withdrawn and the object is removed. Clinicians not infrequently experience difficulty entrapping objects in a basket, often requiring multiple attempts, which lead to prolonged procedure time. Additionally, smaller objects may easily become dislodged from the basket thus requiring the clinician to recapture the object. Further, US 2013/023894 A1 discloses surgical retrieval devices for capturing tissues and fluids in endoscopic and laproscopic operations, US 2016/151080 A1 discloses a body-cavity foreign object capturing device for capturing a foreign object which is produced in a body cavity of the human body, such as a tubular organ, US 2013/131688 A1 discloses a medical device which can be used to resect and remove polyps, stones or foreign bodies, DE 3407708 A1 discloses a probe-like instrument having an elastic guide member for the extraction of urinary calculi from ureters, and US 5944728 A discloses surgical retrieval devices for removing biological or foreign material from the body, having a basket with the ability to capture and release material (e.g., stones, calculi, etc.).

Therefore, an improved system or apparatus is needed that allows for the universal retrieval of target objects from anatomical ducts or lumens, regardless of the size or shape. Such apparatus or system may be capable of reducing the time taken for a clinician to perform an endoscopic procedure and increasing the effectiveness of the procedure.

### SUMMARY

The present invention is directed towards a retrieval device as defined by appended claim 1. Preferred embodiments are defined by the dependent claims.

The embodiments of the present disclosure include devices and methods for retrieving objects in an endoscopic procedure, for example an ERCP procedure. Advantageously, the exemplary embodiments allow for a single instrument to be capable of quickly and effectively retrieving target objects of various sizes and shapes from a body duct or lumen, thereby improving the efficiency and effectiveness of the endoscopic procedure. In some embodiments, the devices and methods disclosed herein allow for the simultaneous retrieval of multiple objects.

According to an exemplary embodiment of the present disclosure, a retrieval device is described. The retrieval device includes a catheter, a strut shaft, a basket, and a device control portion. In some embodiments, the catheter has at least one lumen, a proximal end and a distal end. In some embodiments, the strut shaft has a proximal end and a distal end, and the catheter substantially surrounds the strut shaft. In some embodiments, the catheter can move axially relative to the strut shaft. In some embodiments, the basket is configured to be axially insertable into the catheter lumen. In some embodiments, the basket includes a plurality of struts, each having a proximal and distal end, where the proximal end of each strut is attached to the distal end of the strut shaft and the distal ends form a frame, and a collapsible pouch, where the collapsible pouch is flexibly connected to the distal end of each of the struts. In some embodiments, the device control portion has a proximal end and a distal end, where the distal end is connected to the proximal end of the catheter.

According to a further exemplary embodiment of the present disclosure, a non-claimed method for retrieving one or more objects from a lumen of a patient is described. The method includes providing a retrieval device, inserting the retrieval device into the lumen of the patient, moving the catheter in a proximal direction, entrapping one or more objects in the collapsible pouch, optionally moving the catheter in a distal direction, and withdrawing the retrieval device from the lumen of the patient. In some embodiments, the retrieval device includes a catheter, a strut shaft, a basket, and a device control portion. In some embodiments, the catheter has at least one lumen, a proximal end and a distal end. In some embodiments, the strut shaft has a proximal end and a distal end, and the catheter substantially surrounds the strut shaft. In some embodiments, the catheter can move axially relative to the strut shaft. In some embodiments, the basket is configured to be axially insertable into the catheter lumen. In some embodiments, the basket includes a plurality of struts, each having a proximal and distal end, where the proximal end of each strut is attached to the distal end of the strut shaft and the distal ends form a frame, and a collapsible pouch, where the collapsible pouch is flexibly connected to the distal end of each of the struts. In some embodiments, the device control portion has a proximal end and a distal end, where the distal end is connected to the proximal end of the catheter.

According to a further exemplary embodiment of the present disclosure, a retrieval device is described. The retrieval device includes a catheter, a strut shaft, a basket, and a device control portion, wherein axial movement of the catheter in a proximal direction relative to the strut shaft causes the plurality of struts to spread apart until the collapsible pouch is taut when the basket exits from the distal end of the catheter. In some embodiments, the catheter has at least one lumen, a proximal end, and a distal end. In some embodiments, the strut shaft has a proximal end and distal end, where the catheter substantially surrounds the strut shaft, and where the catheter can move axially relative to the strut shaft. In some embodiments, the basket is configured to be axially insertable into the catheter lumen, and includes a plurality of struts and a collapsible pouch. In some embodiments, each of the plurality of struts has a proximal end and a distal end, where the proximal end of each strut is attached to the distal end of the strut shaft, and the distal ends form a frame. In some embodiments, the collapsible pouch is flexibly connected to the distal end of each of the struts. In some embodiments, the device control portion has a proximal end and a distal end, where the distal end is connected to the proximal end of the catheter.

According to a further exemplary embodiment of the present disclosure a retrieval device is described. The retrieval device includes a catheter having a proximal end and a distal end, a strut shaft having a proximal end and a distal end, a basket, and a device control portion. In some embodiments, the catheter substantially surrounds the strut shaft. In some embodiments, the catheter can move axially relative to the strut shaft. In some embodiments, the basket is configured to be axially insertable into the catheter. In some embodiments, manipulation of the device control portion may result in deployment of the basket. In some embodiments, the basket includes a plurality of struts and a collapsible pouch. In some embodiments, each of the plurality of struts has a proximal end and a distal end, and the proximal end of each strut is attached to the distal end of the strut shaft and the distal ends form a frame. In some embodiments, the collapsible pouch is flexibly connected to the distal end of each of the struts.

Additional features and advantages of the disclosed embodiments will be set forth in part in the description that follows, and in part will be obvious from the description, or may be learned by practice of the disclosed embodiments. The features and advantages of the disclosed embodiments will be realized and attained by the elements and combinations particularly pointed out in the appended claims.

It is to be understood that both the foregoing general description and the following detailed description are exemplary and explanatory only, and are not restrictive of the disclosed embodiments as claimed.

The accompanying drawings constitute a part of this specification. The drawings illustrate several embodiments of the present disclosure and, together with the description, serve to explain the principles of the disclosed embodiments as set forth in the accompanying claims.

### BRIEF DESCRIPTION OF THE DRAWINGS

FIG. 1 is a perspective view of an exemplary device for object retrieval, according to an embodiment of the present disclosure, with a basket in an expanded position.
FIG. 2 is a perspective view of an exemplary device for object retrieval, according to an embodiment of the present disclosure, with a basket in an expanded position.
FIG. 3 is a perspective view of an exemplary device for object retrieval, according to an embodiment of the present disclosure, with a basket in a collapsed position.
FIG. 4 is a perspective view of an exemplary device for object retrieval, according to an embodiment of the present disclosure, with a basket in a collapsed position.
FIG. 5 is a head-on view of an exemplary device for object retrieval, according to an embodiment of the present disclosure, with a basket in an expanded position.
FIG. 6 is a head-on view of an exemplary device for object retrieval, according to an embodiment of the present disclosure, with a basket in an expanded position.
FIG. 7 is a head-on view of an exemplary device for object retrieval, according to an embodiment of the present disclosure, with a basket in a collapsed position.
FIG. 8 is a head-on view of an exemplary device for object retrieval, according to an embodiment of the present disclosure, with a basket in a collapsed position.
FIG. 9 is a close-up view of the distal end of an exemplary device for object retrieval according to an embodiment of the present disclosure, with a basket in an expanded position.
FIG. 10 is a close-up view of the distal end of an exemplary device for object retrieval according to an embodiment of the present disclosure, with a basket in an expanded position.
FIG. 11 is a cross-sectional view of the distal end of an exemplary device for object retrieval according to an embodiment of the present disclosure, with the basket in an expanded position.
FIG. 12 is a cross-sectional view of the distal end of an exemplary device for object retrieval according to an embodiment of the present disclosure, with the basket in an expanded position.
FIG. 13 is a side-view of a basket of an exemplary device for object retrieval according to an embodiment of the present disclosure, with the basket in an expanded position.
FIG. 14 is a perspective view of a basket of an exemplary device for object retrieval according to an embodiment of the present disclosure, with the basket in an expanded position.
FIG. 15 is a perspective view of the distal end of a catheter of an exemplary device for object retrieval according to an embodiment of the present disclosure with the basket in a collapsed position.
FIG. 16 is a perspective view of the distal end of a catheter of an exemplary device for object retrieval according to an embodiment of the present disclosure with the basket in a collapsed position.
FIGS. 17A and 17B are cross-sectional views of the distal end of a catheter of an exemplary device for object retrieval according to an embodiment of the present disclosure.
FIGS. 18A and 18B is a perspective view of a catheter of an exemplary device for object retrieval according to an embodiment of the present disclosure.
FIGS. 19A-19D are perspective views of frames of an exemplary device for object retrieval according to an embodiment of the present disclosure.
FIGS. 20A-20D are perspective views of collapsible pouches of an exemplary device for object retrieval according to an embodiment of the present disclosure.
FIG. 21 is a cross-sectional view of a device control portion of an exemplary device for object retrieval according to an embodiment of the present disclosure.

### DETAILED DESCRIPTION

The disclosed embodiments relate to devices and methods for efficient and effective object removal from an anatomical duct or lumen. Advantageously, embodiments of the present disclosure allow retrieval of one or more objects of various sizes and shape to be removed from a body duct or lumen with a single device.

As used herein, a proximal end may refer to a point or a location along the length of the device of the present disclosure closer to a clinician, e.g., closer to the handle. A distal end may refer to a point or location along the length of the device of the present disclosure further away from a clinician.

According to an aspect of the present disclosure, a retrieval device may comprise a catheter having a proximal and distal end. The catheter may substantially surround a strut shaft, which has a proximal and distal end. In some embodiments, the strut shaft may be sufficiently flexible. In some embodiments, the strut shaft can move axially with respect to the catheter.

In some embodiments, the catheter may have a length ranging from about 150 cm to about 250 cm. In some embodiments, the catheter may have a length of about 200 cm. In some embodiments, the catheter has an outer diameter ranging from about 4 F (about 1.3 mm) to about 12 F (about 4 mm). In some embodiments, the outer diameter of the catheter is about 9 F (about 3 mm). In some embodiments, the catheter has an inner diameter ranging from about 1 mm to about 3.5 mm. In some embodiments, the inner diameter of the catheter is about 2.6 mm. In some embodiments, the catheter is sufficiently flexible such that it may contort to navigate tortuous body lumen. In some embodiments, the catheter may be made of one or more materials chosen from polyurethane, pebax, and polyethylene.

In certain embodiments, the retrieval device may also comprise a basket. This basket may be configured to be axially insertable into a lumen of the catheter. In some embodiments, the basket may comprise a plurality of struts. This plurality of struts forms a frame. In some embodiments, the basket may comprise a collapsible pouch. This collapsible pouch may reside on the frame. The plurality of struts may each have a proximal and distal end, where the proximal end is attached to the distal end of the strut shaft, and the distal end is attached to the collapsible pouch. The distal end of the strut shafts may be attached to the collapsible pouch by a weld, an adhesive, a crimp, a fastener, a rivet, and the like.

In some embodiments, the length of the strut may range from about 10 mm to about 75 mm. In some embodiments, the length of the strut may be about 40 mm. In some embodiments, the width of the strut may range from about 0.25 mm to about 1.25 mm. In some embodiments, the width of the strut may be about 0.75 mm. In some embodiments, the strut may be made of one or more materials chosen from a group including nitinol, surgical grade stainless steel, and titanium.

In some embodiments, the length of the strut shaft may range from about 10 mm to about 75 mm. In some embodiments, the length of the strut shaft may be about 40 mm. In some embodiments, the outer diameter of the strut shaft may range from about 0.75 mm to about 5 mm. In some embodiments, the outer diameter of the strut shaft may be about 1.5 mm. In some embodiments, the strut shaft may have a hollow structure. In some embodiments, the inner diameter of the hollow portion of the strut shaft may range from about 0.5 mm to about 4.75 mm. In some embodiments, the inner diameter of the hollow portion of the strut shaft may be about 1.25 mm. In some embodiments, the strut shaft may be made of one or more materials chosen from a group including nitinol, surgical grade stainless steel, and the like.

In some embodiments, the collapsible pouch has a diameter ranging from about 5 mm to about 50 mm. In some embodiments, the diameter may be about 20 mm. In some embodiments, the depth of the pouch may range from about 1 mm to about 15 mm. In some embodiments, the depth of the pouch may be about 7 mm.

In some embodiments, the collapsible pouch may be a film, mesh, netting, or other suitable structure. In some embodiments, the collapsible pouch may be a biocompatible film. In some embodiments, the film may be made of one or more biocompatible materials chosen from polyurethane, polypropylene, and polyethylene. In some embodiments, the collapsible pouch may be a mesh. In some embodiments, the mesh may be made of one or more materials chosen from nitinol, surgical grade stainless steel, and polyester.

In certain embodiments, the collapsible pouch has a guidewire port. The guidewire port may, in some embodiments, facilitate use of the device for object retrieval of the present disclosure in cooperation with a guidewire. In some embodiments, the guidewire port may have a diameter ranging from about 0.025" (about 0.63 mm) to about 0.050" (about 1.27 mm). In some embodiments, the guidewire port may have a diameter of about 0.040" (about 1.0 mm).

In other embodiments, the retrieval device may also comprise a device control portion. The device control portion may have a proximal end and a distal end, where the distal end is attached to the proximal end of the catheter. In some embodiments, the device control portion is chosen from a handle, robotic driver, and the like. In some embodiments, the device control portion further comprises an actuation mechanism. In some embodiments, the actuation mechanism may control deployment and retraction of the basket. Exemplary actuation mechanisms include, e.g., a thumb slide, lever, ratchet, rotatable knob or collar, finger rings, etc.

In certain embodiments, an exemplary device control portion is a handle. In some embodiments, the handle may further comprise a stationary handle piece. The stationary handle piece may have a proximal end and a distal end. In some embodiments, the handle may have one or more finger rings, for example, one, two, three, four, or five finger rings. In certain embodiments, the handle may have one finger ring. In other embodiments, the handle may further comprise a moveable handle piece. In some embodiments, the moveable handle piece may have one or more finger rings, for example, one, two, three, four, or five finger rings. In some embodiments, the moveable handle piece may have two finger rings.

In some embodiments, the handle may have an overall length ranging from about 4" (about 10 cm) to about 12" (about 30 cm). In some embodiments, the handle has an overall length of about 9" (about 23 cm). In some embodiments, the handle has an outer diameter ranging from about 0.4" (about 1.0 cm) to about 1" (about 2.5 cm). In some embodiments, the handle has an outer diameter of about 0.63" (about 1.65 cm). In some embodiments, the handle may be made of one or more materials chosen from a group including ABS, polypropylene, and polyethylene.

In some embodiments, the actuation mechanism, e.g., a moveable handle piece, advantageously controls movement of the catheter and the wedge shaft. In some embodiments, the actuation mechanism, e.g., a moveable handle piece, advantageously controls movement of the strut shaft. As the skilled artisan will appreciate, the moveable handle piece could be replaced with, for example, a lever, a slide, a button, a switch, a knob, or the like.

Movement of the moveable handle piece from the distal position to the proximal position may sequentially cause the catheter to move in a proximal direction, thereby exposing the collapsed basket and allowing it to expand, and, in some embodiments, moving the wedge shaft in a proximal direction, thereby jamming the wedge against the proximal end of each strut causing the collapsible pouch to achieve a maximum pouch diameter and forcing the struts apart to maximize the space in between each of them, known as an expanded position. Without being bound by any theory, the jamming of the wedge against the proximal end of each strut to achieve a maximum pouch diameter may aid in maximizing the number of objects collected in the collapsible pouch. In other embodiments, movement of the moveable handle piece from the proximal position to the distal position may sequentially cause the wedge shaft to move in a distal direction, and the catheter to move in a distal direction, thereby collapsing and then substantially surrounding the basket, known as a collapsed position. However, this description is merely exemplary and the skilled artisan will appreciate that any configuration may be suitable, for example, the catheter may remain stationary and the strut shaft may move axially relative to the catheter in a distal direction, thereby exposing the collapsed basket and allowing it to expand.

In some embodiments, the retrieval device may further comprise a wedge shaft, having a proximal end and a distal end. In some embodiments, the wedge shaft may be configured to be substantially surrounded by the strut shaft. In some embodiments, the wedge shaft may be configured to be partially surrounded by the strut shaft. In other embodiments, the retrieval device may further comprise a wedge. In some embodiments, the wedge may be attached to the distal end of the wedge shaft.

In some embodiments, the wedge may be spherical. In some embodiments, the wedge may be rectangular. In some embodiments, the wedge may be conical. In some embodiments, the wedge may have a width ranging from about 0.040" (about 1 mm) to about 0.120" (about 3 mm). In some embodiments, the wedge may have a width of about 0.075" (about 1.9 mm). In some embodiments, the wedge may have a length ranging from about 0.040 (about 1 mm) to about 0.160" (about 4 mm). In some embodiments, the wedge may have a length of about 0.060" (about 1.5 mm). In some embodiments, the wedge may have a hollow structure. In some embodiments, the hollow portion of the wedge may be configured to receive a guidewire. In some embodiments, the inner diameter of the wedge having a hollow structure may have a range from about 0.075 mm (about 0.030") to about 1.5 mm (about 0.060"). In some embodiments, the inner diameter of the wedge having a hollow structure may be about 1 mm (about 0.040"). In some embodiments, the wedge may be made of one or more materials chosen from polyimide, peek, and polyethylene.

In some embodiments, the wedge shaft may have a length ranging from about 150 cm to about 250 cm. In some embodiments, the wedge shaft may have a length of about 200 cm. In some embodiments, the wedge shaft may have a solid structure with an outer diameter ranging from about 0.25 mm (about 0.010") to about 0.75 mm (about 0.030"). In some embodiments, the wedge shaft may have an outer diameter of about 0.4 mm (about 0.016"). In some embodiments, the wedge shaft may have a hollow structure. In some embodiments, the inner-diameter of the wedge shaft having a hollow structure may range from about 0.75 mm (about 0.030") to about 1.5 mm (about 0.060"). In some embodiments, the inner-diameter of the wedge shaft having a hollow structure may be about 1.1 mm. In some embodiments, the outer diameter of the wedge shaft having a hollow structure may range from about 0.9 mm (about 0.35") to about 2 mm (about 0.080"). In some embodiments, the wedge shaft may be made of one or more materials chosen from polyimide, peek, and polyethylene. In some embodiments, the wedge shaft may be reinforced with, e.g., a stainless steel wire braid or coil. In some embodiments, friction reducing linings, e.g., PTFE, may be applied on the inside and outside surfaces.

In some embodiments, the catheter may further comprise a guidewire lumen. The guidewire lumen is configured to receive a guidewire. For example, guidewire lumen may extend over a predetermined length of the catheter. A guidewire may be inserted and passed through guidewire lumen. In some embodiments, the guidewire lumen may have a slit extending over at least a portion of its length.

In some embodiments, the wedge shaft may be solid. In some embodiments, the wedge shaft may have a hollow structure. In some embodiments, the hollow portion of the wedge shaft may be centered in the wedge shaft. In other embodiments, the hollow portion of the wedge shaft may be off-center in the wedge shaft. In some embodiments, the hollow portion of the wedge shaft may be configured to receive a guidewire.

If a clinician determines that a person may have an object in a lumen, they may use a retrieval device of the present disclosure. While the basket is in a collapsed position, the clinician may insert the retrieval device to the site of the object. This may be accomplished by use of a guidewire or use of another instrument to visualize the object, e.g., an endoscope. Upon reaching the site of the object, the clinician may then desire to deploy the basket. This may be achieved by manipulation of the actuation mechanism, e.g., a handle, by the clinician, e.g., by moving a moveable handle piece or a lever to a proximal position, which causes the basket (collapsible pouch and struts) to achieve an expanded position. A withdrawal motion of the retrieval device may cause the object to become entrapped in the collapsible pouch. In some embodiments, the clinician may opt to collapse the pouch to facilitate withdrawal by, e.g., manipulating the actuation mechanism. In other embodiments, the clinician may opt to partially withdraw the retrieval device with the basket in an expanded position, e.g., to attempt to retrieve more objects as the retrieval device is withdrawn.

Reference will now be made in detail to embodiments and aspects of the present disclosure, examples of which are illustrated in the accompanying drawings. Where possible, the same reference numbers will be used throughout the drawings to refer to the same or like parts.

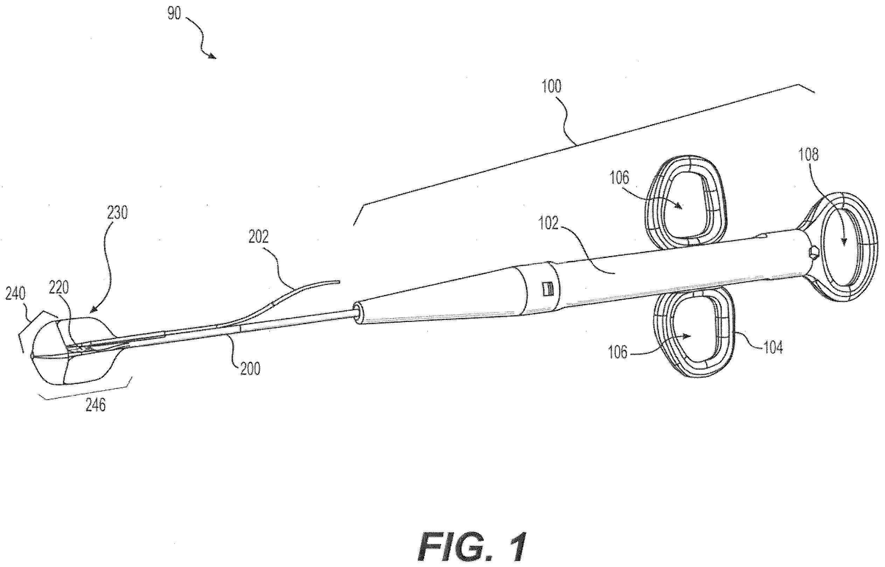

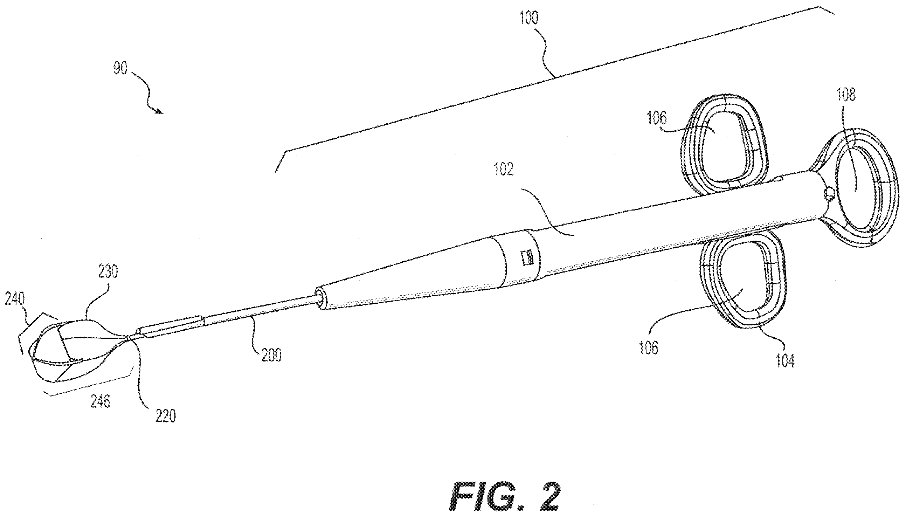

FIG. 1 is a perspective view of a device 90 for object retrieval according to an exemplary embodiment of the present disclosure. As shown in FIG. 1, device 90 may include an actuation mechanism, wherein the actuation mechanism includes a handle 100, a catheter 200, and a basket 246. Handle 100 may further include a stationary handle piece 102, a moveable handle piece 104, and finger rings 106 and 108. The clinician can actuate movement of the distal retrieval end of the device by moving finger rings 106 and 108. Of course, this arrangement is merely exemplary and any type of actuation can be employed, such as thumb slide, lever, ratchet, rotatable knob or collar, etc. Basket 246 may further include a plurality of struts 230, for example, two, three, four, five, six, or more struts, and a collapsible pouch 240. Catheter 200 may substantially surround a wedge shaft (not shown) which comprises a wedge 220 attached at its distal end. Additionally, catheter 200 may be configured to receive a guidewire 202, via, e.g., a guidewire lumen (not shown). In some embodiments, no guidewire is present, as shown in FIG. 2. As shown in FIG. 1, basket 246 is in an expanded position.

In some embodiments, manipulation of handle 100, e.g., by movement of moveable handle piece 104, e.g., from a distal position to a proximal position, may result in a compound action that results in deployment and expansion of basket 246.

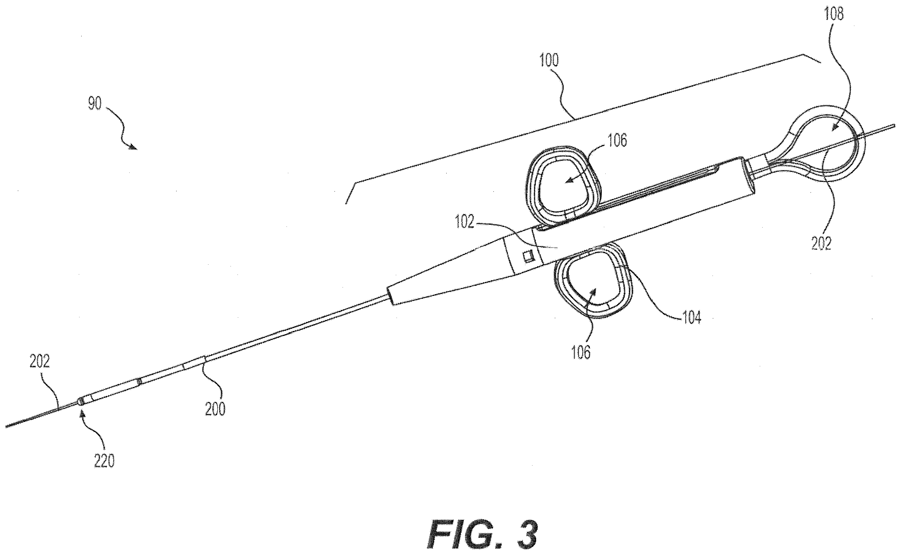

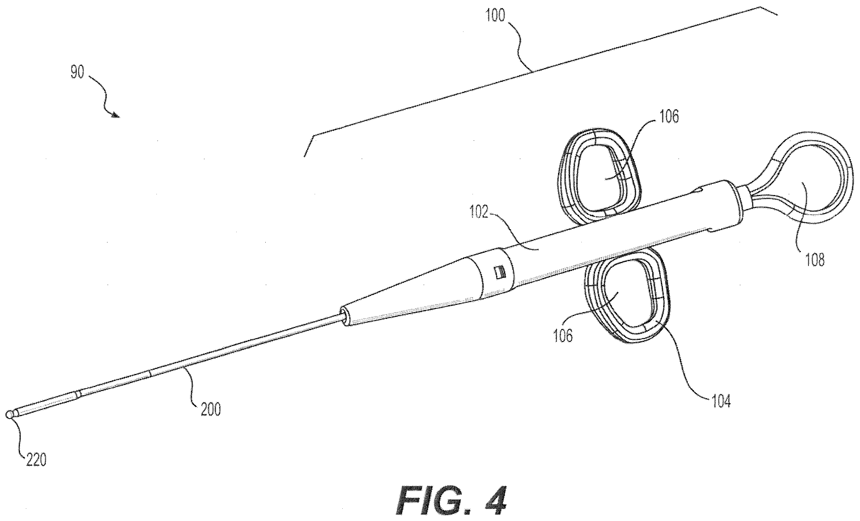

FIG. 3 is a perspective view of a device 90 for object retrieval according to an exemplary embodiment of the present disclosure. As shown, the basket (not shown) is in a collapsed position and has been retracted and is substantially surrounded by catheter 200. In some embodiments, no guidewire is present, as shown in FIG. 4.

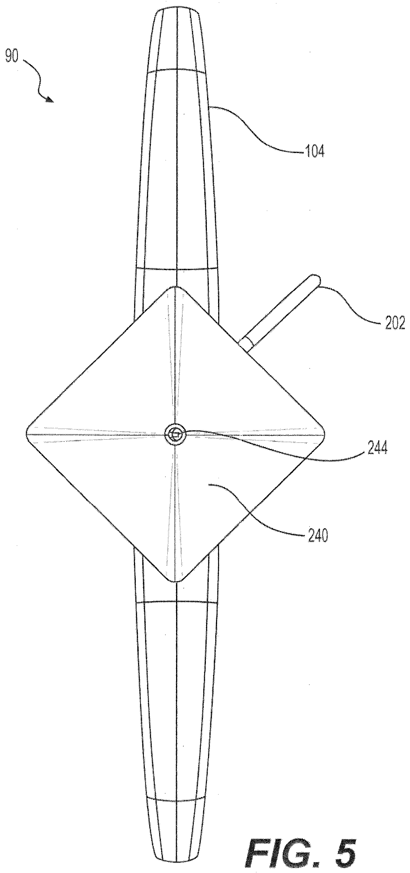

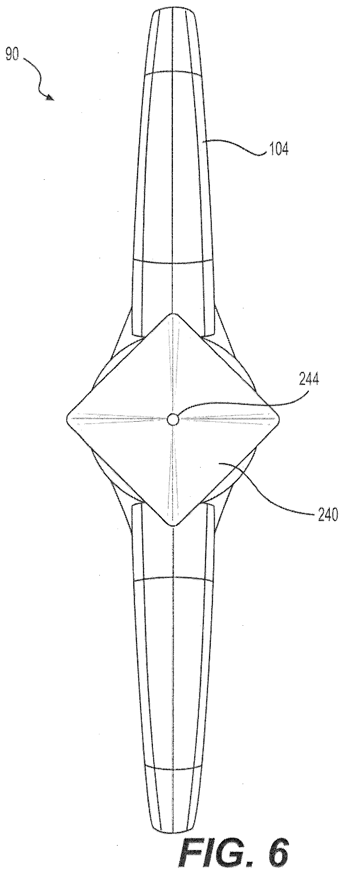

FIG. 5 is a head-on view of device 90 for object retrieval according to an exemplary embodiment of the present disclosure. A guidewire port 244 allows the device to be inserted and withdrawn making use of guidewire 202, but without interfering with the position of guidewire 202. In some embodiments, no guidewire is present, as shown in FIG. 6. In some embodiments, guidewire 202 is present and extends alongside basket 246 (not shown).

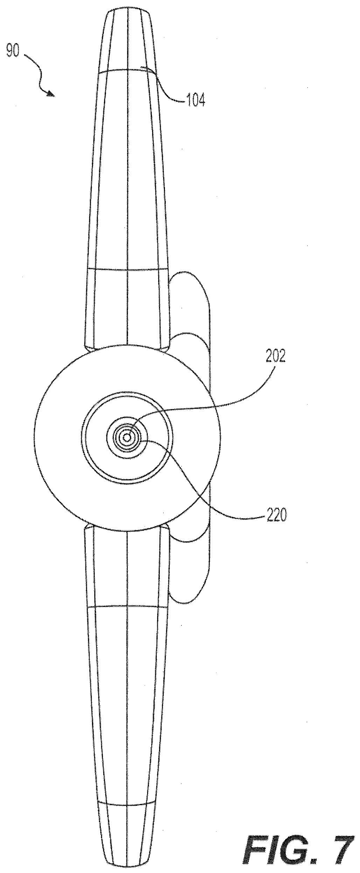

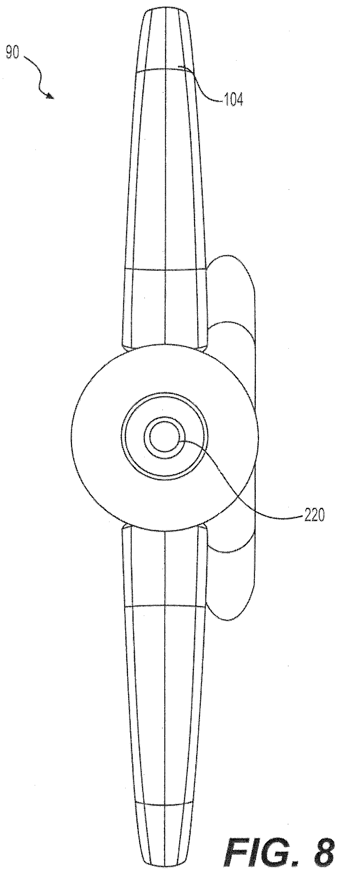

FIG. 7 is a head-on view of device 90 for object retrieval according to an exemplary embodiment of the present disclosure. Here, guidewire 202 can be seen in the center of wedge 220. As will be appreciated by the skilled artisan, guidewire 202 can be centered or off-center in wedge 220. In some embodiments, no guidewire is present, as shown in FIG. 8.

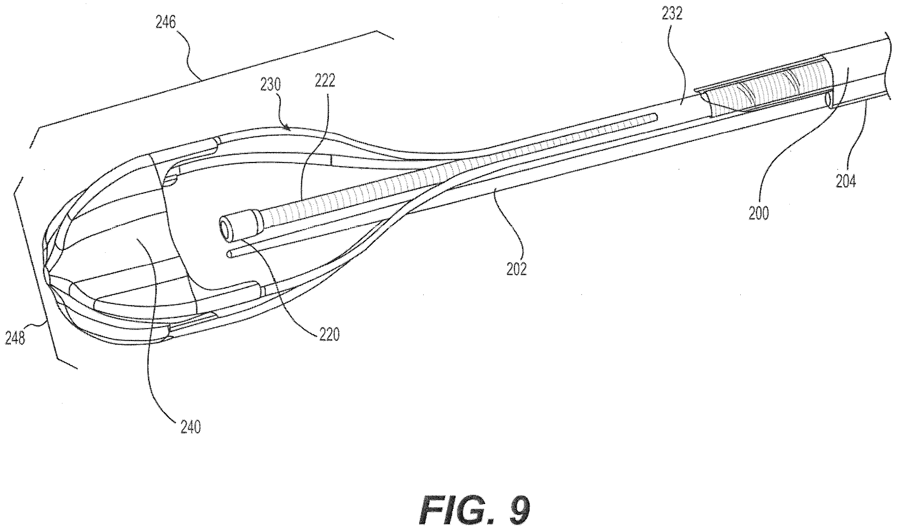

FIG. 9 is a close-up view of the distal end of device 90 for object retrieval in accordance with the present invention. As shown, basket 246 is in an expanded position. The distal end of each of struts 230 is attached to collapsible pouch 240, whereas the proximal end of each of struts 230 is attached to a strut shaft 232. Strut shaft 232 is substantially surrounded by catheter 200. A wedge shaft 222 has a proximal end and distal end; attached to the distal end of wedge shaft 222 is a wedge 220. Wedge shaft 222 may be moved distally with respect to catheter 200 by, e.g., manipulation of handle 100 (not shown). Such a movement may cause wedge 220 to jam against the each of the proximal ends of struts 230, thereby forcing struts 230 to spread further apart, thus causing basket 246 to achieve a maximum opening diameter 248. In some embodiments, this configuration may aid in entrapping one or more objects present in a lumen.

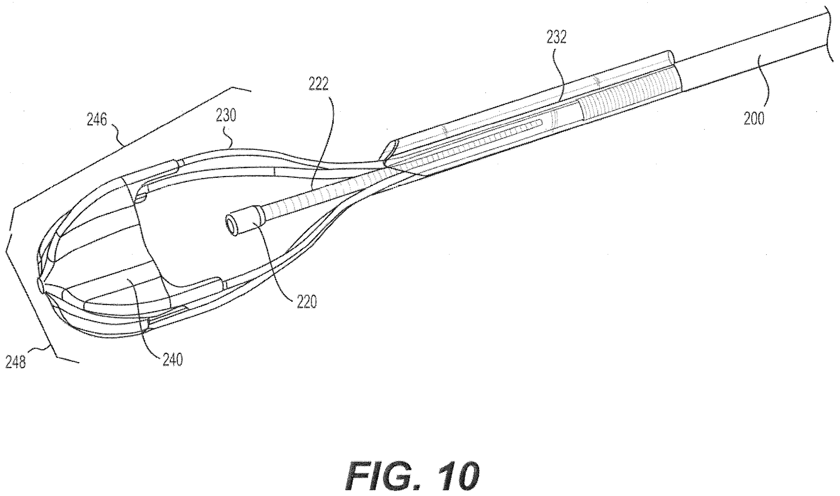

In some embodiments, guidewire 202 may optionally be present. As shown in FIG. 9, catheter 200 is configured to receive guidewire 202 via a guidewire lumen 204. In other embodiments, wedge 220 and wedge shaft 222 may have a hollow inner-diameter which can be configured to receive a guidewire 202. In other embodiments, not shown here, wedge 220 and wedge shaft 222 are not hollow, and thus are not configured to receive guidewire 202. In other embodiments, no guidewire is present, as shown in FIG. 10.

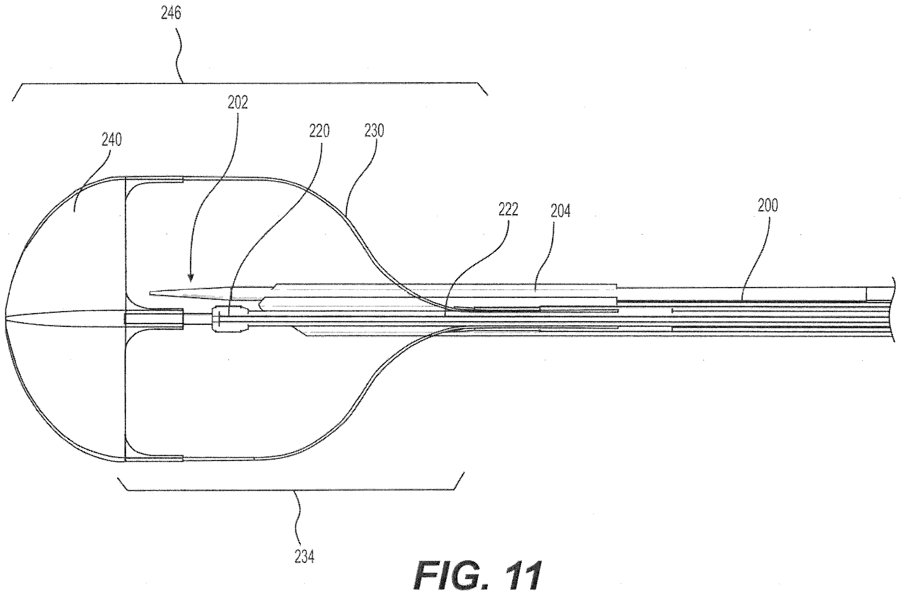

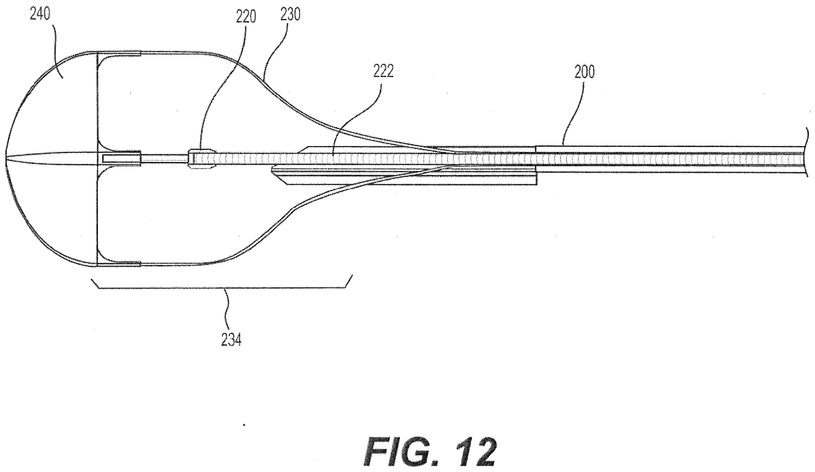

FIG. 11 is a cross-sectional view of the distal end of device 90 for object retrieval according to an exemplary embodiment of the present disclosure. This figure shows a cross-sectional view of the exemplary device of FIG. 5. Here, it can be seen how catheter 200 may be configured to receive a guidewire 202 via guidewire lumen 204. In other embodiments, wedge 220 and wedge shaft 222 may have hollow structures, where the hollow portions may be configured to receive guidewire 202. Struts 230 may cooperatively form a frame 234, upon which collapsible pouch 240 resides by way of attachment to each of the distal ends of struts 230. In some embodiments, no guidewire is present, as shown in FIG. 12.

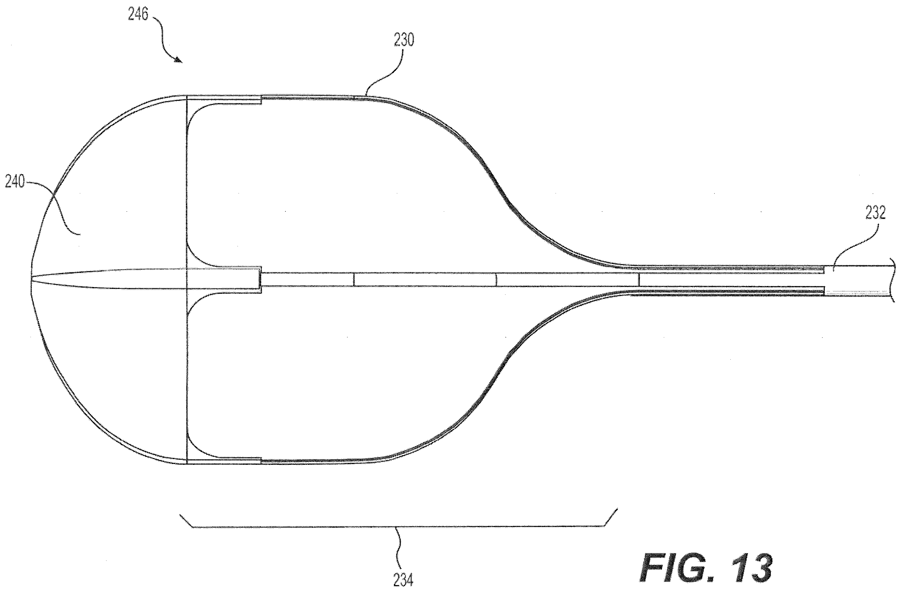

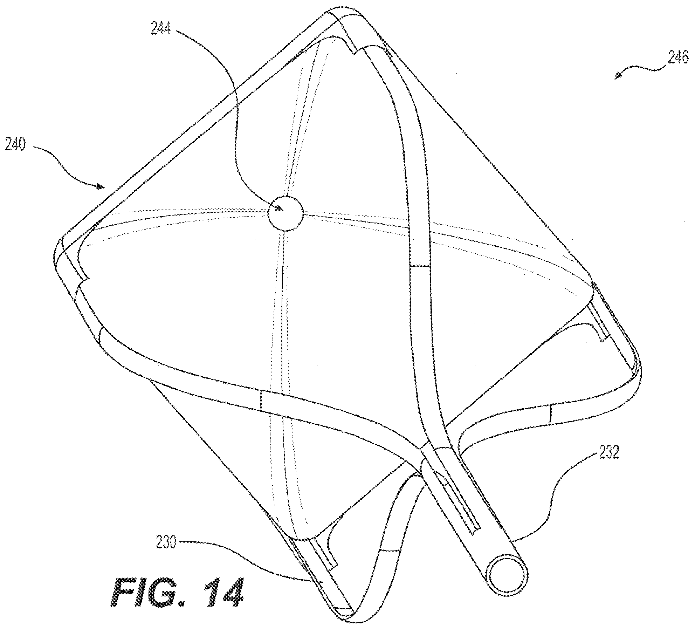

FIG. 13 is a side-view of basket 246. Basket 246 includes a plurality of struts 230, where the proximal end of each of struts 230 is attached to the distal end of a strut shaft 232. Struts 230 cooperatively make up frame 234, upon which collapsible pouch 240 resides. Each of struts 230 are attached to collapsible pouch 240 by way of an attachment point 242. Struts 230 are formed in such a way that, when in their relaxed state, they assume a position that is more expanded than in a collapsed state. FIG. 14 is an alternative, perspective view of FIG. 13.

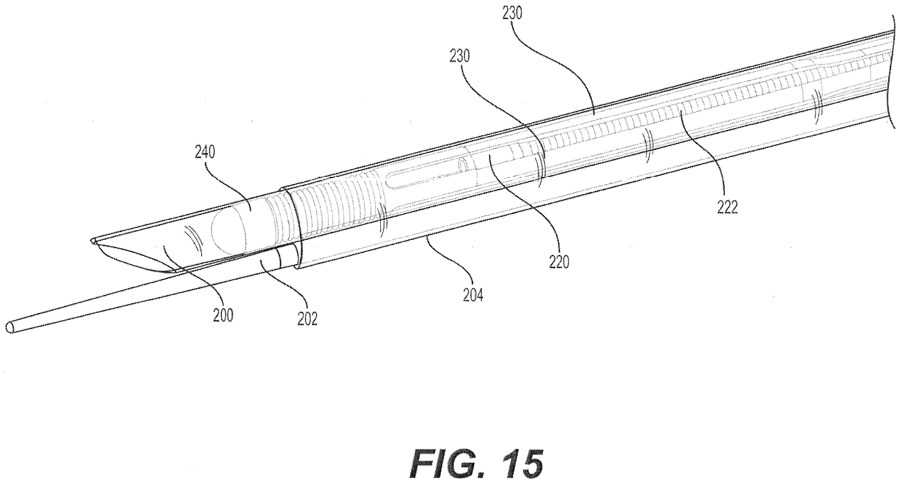

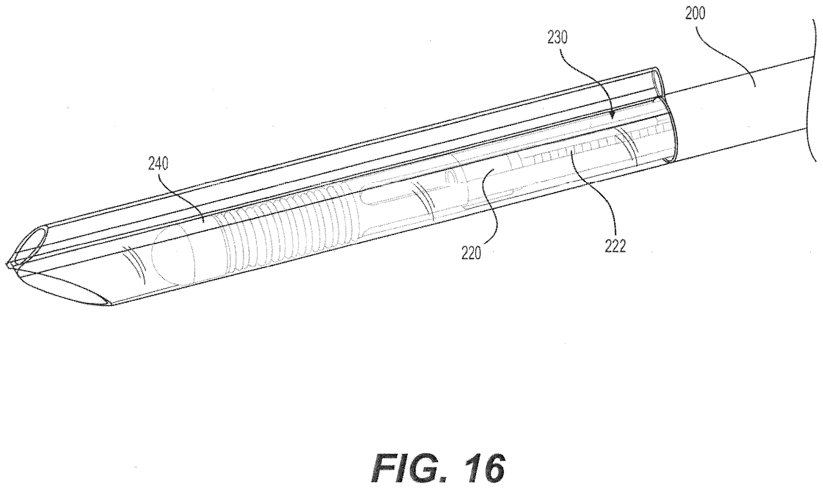

FIG. 15 shows a perspective view of the distal end of a catheter 200 of device 90 for object retrieval according to an exemplary embodiment of the present disclosure. Here, the basket (not shown) formed by struts 230 and collapsible pouch 240 is in a collapsed position and is substantially surrounded by catheter 200. Wedge 220 and wedge shaft 222 are substantially surrounded by struts 230 and collapsible pouch 240. Catheter 200 has been configured to receive guidewire 202 by way of guidewire lumen 204. In some embodiments, no guidewire is present. In other embodiments, wedge 220 and wedge shaft 222 each have a hollow structure which may be configured to receive a guidewire. In some embodiments, no guidewire is present, as shown in FIG. 16.

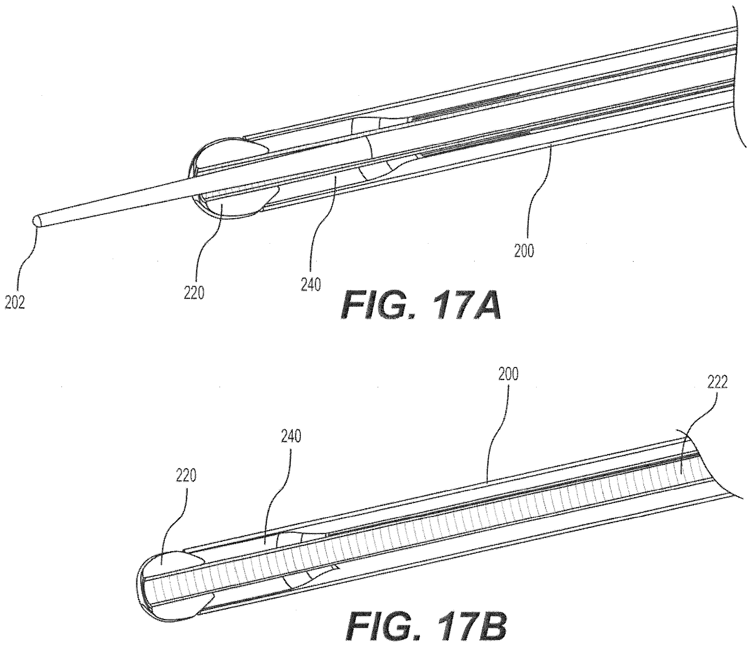

FIGS. 17A and 17B show cross-sectional views of the distal end of catheter 200 of a device for object retrieval according to an exemplary embodiment of the present disclosure. Both FIGS. 17A and 17B show the inner-diameter of the hollow portions of wedge 220 and wedge shaft 222. In some embodiments, *e.g.,* FIG. 17A, the inner-diameter of the hollow portion of wedge 220 and wedge shaft 222 may be configured to receive a guidewire. In other embodiments, *e.g.,* FIG. 17B, no guidewire is present in the hollow portion of wedge 220 and wedge shaft 222. In both FIGS. 17A and 17B, collapsible pouch 240 is in a collapsed position and is substantially surrounded by catheter 200.

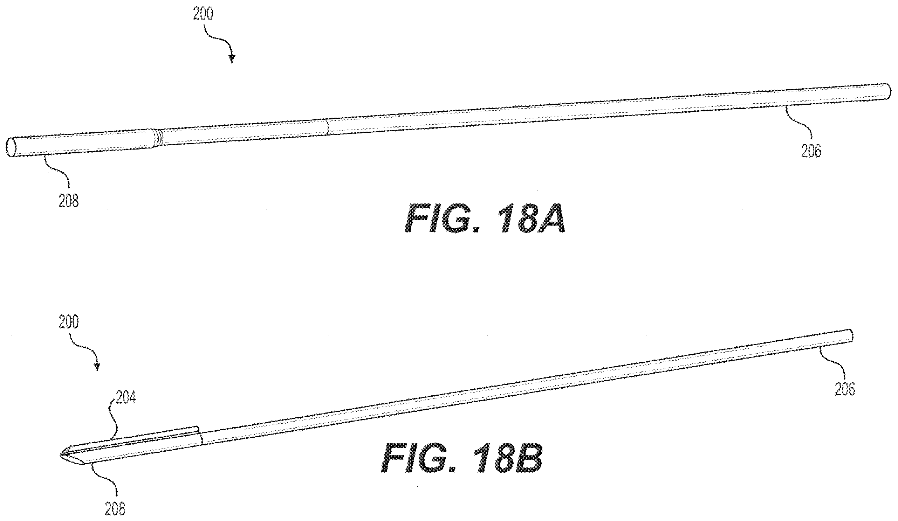

FIGS. 18A and 18B are perspective views of catheter 200 of device 90 for object retrieval according to an exemplary embodiment of the present disclosure. As shown, catheter 200 has a proximal end 206 and a distal end 208. In some embodiments, proximal end 206 may be attached to a device control portion, *e.g.,* a handle. In some embodiments, catheter 200 may be configured to receive a guidewire via guidewire lumen 204, as sown in FIG. 18B.

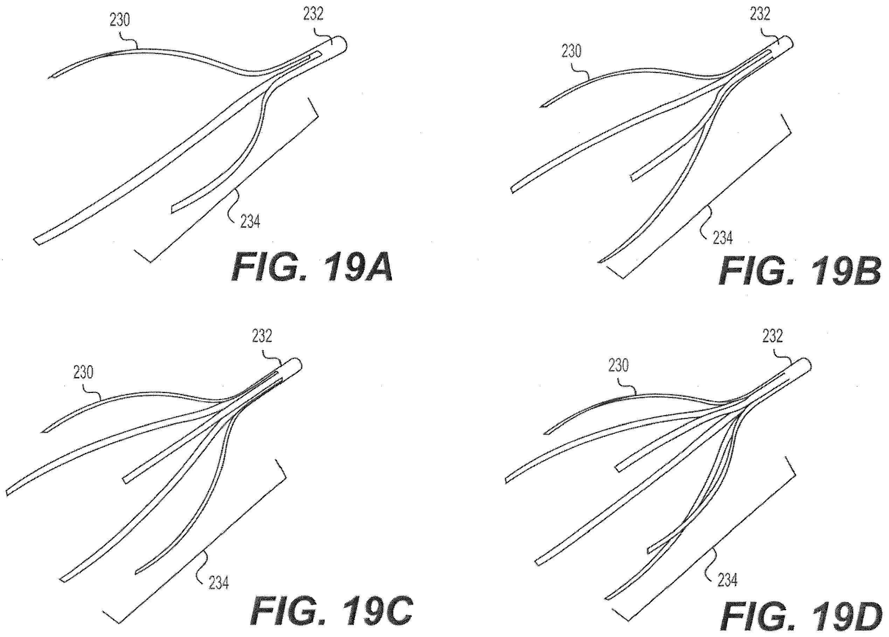

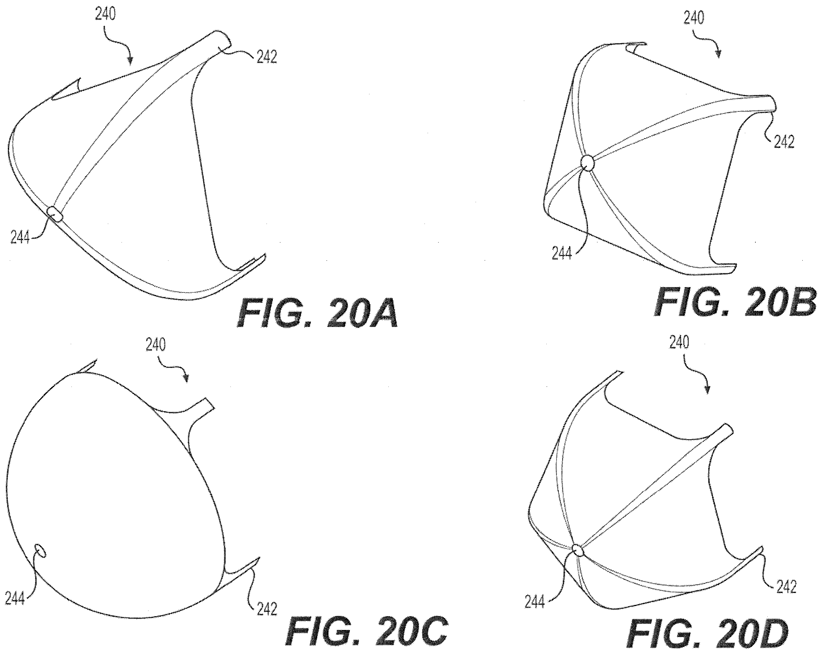

FIGS. 19A-19D are perspective views of frames of an exemplary device 90 for object retrieval. In some embodiments, there may be one or more struts 230, *e.g.,* one, two, three, four, five, six, seven, eight, nine, ten, or more struts 230. In some embodiments, *e.g.,* FIG. 19A, frame 234 has three struts 230. In some embodiments, *e.g.,* FIG. 19B, frame 234 has four struts 230. Some illustrative embodiments, such as that shown in FIG. 19C, have five struts 230. In some embodiments, *e.g.,* FIG. 19D, frame 234 has six struts 230. In some embodiments, the proximal end of each strut 230 is attached to the distal end of strut shaft 232. In some embodiments, the distal end of each strut 230 is attached to an attachment point (see FIGS. 20A-20D) of collapsible pouch 240 (not shown).

FIGS. 20A-20D are perspective views of embodiments of collapsible pouch 240 of an exemplary device 90 for object retrieval. As shown, collapsible pouch 240 has a guidewire port 244 and one or more attachment points 242. In certain embodiments, collapsible pouch 240 has one or more attachment points, *e.g.,* one, two, three, four, five, six, seven, eight, nine, ten, or more attachment points 242. In some embodiments, *e.g.,* as shown in FIG. 20A, collabsible pouch 240 has three attachment points 242. In some embodiments, *e.g.,* as shown in FIGS. 20B and 20C, collapsible pouch 240 has four attachment points 242. In some embodiments, *e.g.,* as shown in FIG. 20D, collapsible pouch 240 has five attachment points 242.

Collapsible pouch 240 may assume a wide variety of geometric shapes. In some embodiments, *e.g.,* as shown in FIG. 20C, collapsible pouch 240 assumes a bowl-like shape. In other embodiments, *e.g.,* as shown in FIGS. 20B and 20D, collapsible pouch 240 assumes an umbrella-like shape.

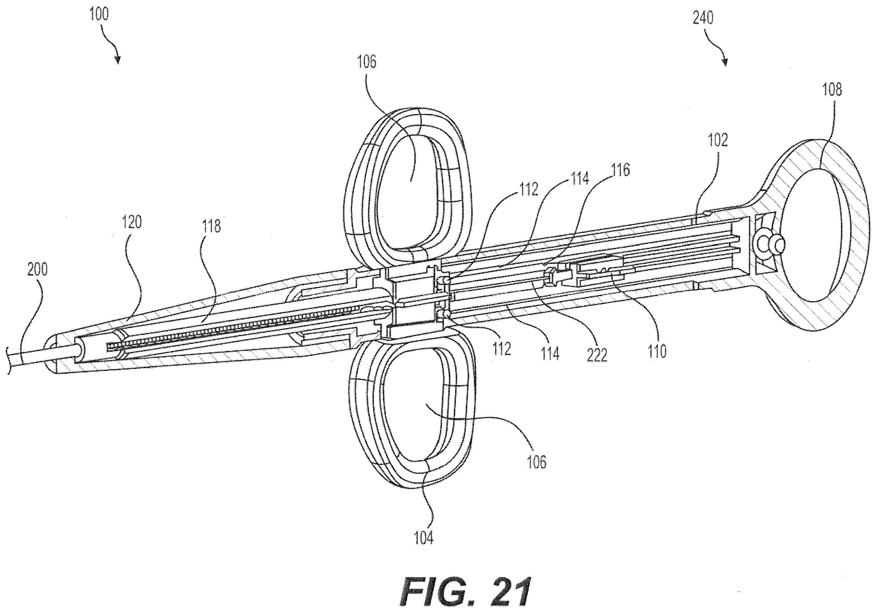

FIG. 21 is a perspective, cross-sectional view of the control portion of an exemplary device 90 , according to an exemplary embodiment of the present disclosure. As shown in FIG. 21, the control portion includes a handle 100. As described above, handle 100 includes a stationary handle piece 102, a moveable handle piece 104, and finger rings 106 and 108. Catheter 200 is coupled to moveable handle piece 104 via a catheter coupling piece 118 such that catheter 200 moves with movement of handle piece 104. Catheter coupling piece 118 is covered by catheter coupling piece cover 120.

Moveable handle piece 104 moves axially along one or more cams 114 and is guided by one or more engagement blocks 112, resulting in deployment of a basket from the distal end of catheter 200. In one embodiment, axial movement of handle piece 104 in proximal direction causes catheter 200 to move in a proximal direction. When moveable handle piece 104 is moved to a cam transition point 116, engagement block 112 engages with wedge slide 110. Wedge slide 110 is coupled to wedge shaft 222. Upon engagement, axial movement in a proximal direction of moveable handle piece 104 causes axial movement in a proximal direction of wedge slide 110 and wedge shaft 222. This movement causes wedge 220 (not shown), located at the distal end of wedge shaft 222, to jam against the proximal end of the struts 230 (not shown), thus forcing them to spread apart.

Closing and retraction of basket 246 occurs in the opposite order of the aforementioned process. Axial movement of moveable handle piece 104 in a distal direction results in an unjamming of a wedge at the end of wedge shaft 222 by axial movement in a distal direction. Further axial movement in a distal direction of moveable handle piece 104 results in disengagement of engagement block 112 from wedge slide 110, followed by collapse of the basket 246 (not shown) and retraction of the basket 246 into the distal end of catheter 200 by axial movement of catheter 200 in a distal direction. The skilled artisan, will, however, appreciate that this configuration is merely exemplary and deployment of the basket can be effected by axial movement of strut shaft 232 while catheter 200 remains stationary.

The foregoing description has been presented for purposes of illustration. It is not exhaustive and is not limited to precise forms or embodiments disclosed. Modifications and adaptations of the embodiments will be apparent from consideration of the specification and practice of the disclosed embodiments. For example, the described implementations include hardware and software, but systems and methods consistent with the present disclosure can be implemented as hardware alone. In addition, while certain components have been described as being coupled to one another, such components may be integrated with one another or distributed in any suitable fashion.

Moreover, while illustrative embodiments have been described herein, the scope includes any and all embodiments having equivalent elements, modifications, omissions, combinations (*e.g.*, of aspects across various embodiments), adaptations and/or alterations based on the present disclosure. The elements in the claims are to be interpreted broadly based on the language employed in the claims and not limited to examples described in the present specification or during the prosecution of the application, which examples are to be construed as nonexclusive. Further, the steps of the disclosed methods can be modified in any manner, including reordering steps and/or inserting or deleting steps.

## Claims

1. A retrieval device, comprising:
a catheter (200) having at least one catheter lumen, a proximal end and a distal end;
a strut shaft (232) having a proximal end and a distal end, wherein the catheter (200) substantially surrounds the strut shaft (232), and wherein the catheter (200) can move axially relative to the strut shaft (232):
a basket (246), wherein the basket (246) is configured to be axially insertable into the catheter lumen, and comprising:
a plurality of struts (230), each having a proximal end and a distal end, wherein the proximal end of each strut (230) is attached to the distal end of the strut shaft (232) and the distal ends of the struts (230) form a frame; and
a collapsible pouch (240) , wherein the collapsible pouch (240) is flexibly connected to the distal end of each of the struts (230); and
a device control portion having a proximal end and a distal end, wherein the distal end is connected to the proximal end of the catheter (200), and the retrieval device further comprising:
a wedge shaft (222) having a proximal end and a distal end;
a wedge (220); and
a wedge slide (110) coupled to the wedge shaft (222),
wherein:
the wedge (220) is attached to the distal end of the wedge shaft (222); and the strut shaft (232) substantially surrounds the wedge shaft (222),
wherein
the wedge (220) located at the distal end of the wedge shaft (222) is configured to be jammed against the proximal end of the struts (230) and the struts (230) are spread apart, by axial movement in a proximal direction of the wedge slide (110) and the wedge shaft (222).

2. The retrieval device according to claim 1,
wherein the device control portion is a handle (100).

3. The retrieval device according to claim 2,
wherein the handle (100) further comprises:
a stationary handle piece (102) having a proximal end and a distal end, and having one or more finger rings (106, 108); and
a moveable handle piece (104) having one or more finger rings (106, 108),
wherein:
the moveable handle piece (104) is configured to move axially relative to the stationary handle piece (102).

4. The retrieval device according to claim 3, wherein the proximal end of the strut shaft (232) is coupled to the distal end of the stationary handle piece (102).

5. The retrieval device according to claim 3,
wherein the proximal end of the catheter (200) is coupled to the moveable handle piece, and wherein the moveable handle piece (104) is configured to move the catheter (200) axially relative to the strut shaft (232).

6. The retrieval device according to claim 3,
wherein the proximal end of the catheter (200) and the proximal end of the wedge shaft (222) are coupled to the moveable handle piece (104), and wherein the moveable handle piece (104) is configured to move the catheter (200) and the wedge shaft (222) axially relative to the strut shaft (232).

7. The retrieval device according to claim 1.
wherein when the catheter (200) is in a proximal most position, the struts (230) are in an expanded position, and when the catheter (200) is in a distal most position, the struts (230) are in a collapsed position and are substantially surrounded by the catheter (200).

8. The retrieval device according to claim 1.
wherein the catheter (200) further comprises a guidewire lumen (204).

9. The retrieval device according to claim 1,
wherein the wedge shaft (222) has a hollow portion.

10. The retrieval device according to claim 9,
wherein the hollow portion of the wedge shaft (222) is configured to receive a guidewire (202).

11. The retrieval device according to claim 1,
wherein the collapsible pouch (240) is a film or a mesh.

12. The retrieval device according to claim 1,
wherein when the basket (246) exits from the distal end of the catheter (200), axial movement of the catheter (200) in a proximal direction relative to the strut shaft (232) causes the plurality of struts (230) to spread apart until the collapsible pouch is taut.

## Patentansprüche

1. Rückholvorrichtung, umfassend:
einen Katheter (200), der mindestens ein Katheterlumen, ein proximales Ende und ein distales Ende aufweist;
eine Verstrebungswelle (232), die ein proximales Ende und ein distales Ende aufweist, wobei der Katheter (200) die Verstrebungswelle (232) im Wesentlichen umgibt, und wobei der Katheter (200) sich relativ zu der Verstrebungswelle (232) axial bewegen kann;
einen Korb (246), wobei der Korb (246) dazu konfiguriert ist, axial in das Katheterlumen einführbar zu sein, und Folgendes umfasst:
eine Vielzahl von Verstrebungen (230), wobei jede ein proximales Ende und ein distales Ende aufweist, wobei das proximale Ende jeder Verstrebung (230) an dem distalen Ende der Verstrebungswelle (232) angebracht ist und die distalen Enden der Verstrebungen (230) einen Rahmen ausbilden; und
einen zusammenlegbaren Beutel (240), wobei der zusammenlegbare Beutel (240) flexibel mit dem distalen Ende jeder der Verstrebungen (230) verbunden ist; und
einen Vorrichtungssteuerabschnitt, der ein proximales Ende und ein distales Ende aufweist, wobei das distale Ende mit dem proximalen Ende des Katheters (200) verbunden ist, und die Rückholvorrichtung ferner Folgendes umfasst:
eine Keilwelle (222), die ein proximales Ende und ein distales Ende aufweist;
einen Keil (220); und
einen Keilschieber (110), der mit der Keilwelle (222) gekoppelt ist,
wobei:
der Keil (220) an dem distalen Ende der Keilwelle (222) angebracht ist; und die Verstrebungswelle (232) die Keilwelle (222) im Wesentlichen umgibt,
wobei
der an dem distalen Ende der Keilwelle (222) angeordnete Keil (220) dazu konfiguriert ist, gegen das proximale Ende der Verstrebungen (230) gedrückt zu sein und die Verstrebungen (230) durch eine axiale Bewegung in einer proximalen Richtung des Keilschiebers (110) und der Keilwelle (222) auseinander gespreizt werden.

2. Rückholvorrichtung nach Anspruch 1,
wobei der Vorrichtungssteuerabschnitt ein Griff (100) ist.

3. Rückholvorrichtung nach Anspruch 2,
wobei der Griff (100) ferner Folgendes umfasst:
ein stationäres Griffteil (102), das ein proximales Ende und ein distales Ende aufweist und das einen oder mehrere Fingerringe (106, 108) aufweist; und
ein bewegliches Griffteil (104), das einen oder mehrere Fingerringe (106, 108) aufweist,
wobei:
das bewegliche Griffteil (104) dazu konfiguriert ist, sich relativ zu dem stationären Griffteil (102) axial zu bewegen.

4. Rückholvorrichtung nach Anspruch 3,
wobei das proximale Ende der Verstrebungswelle (232) mit dem distalen Ende des stationären Griffteils (102) gekoppelt ist.

5. Rückholvorrichtung nach Anspruch 3,
wobei das proximale Ende des Katheters (200) mit dem beweglichen Griffteil gekoppelt ist, und wobei das bewegliche Griffteil (104) dazu konfiguriert ist, den Katheter (200) relativ zu der Verstrebungswelle (232) axial zu bewegen.

6. Rückholvorrichtung nach Anspruch 3,
wobei das proximale Ende des Katheters (200) und das proximale Ende der Keilwelle (222) mit dem beweglichen Griffteil (104) gekoppelt sind, und wobei das bewegliche Griffteil (104) dazu konfiguriert ist, den Katheter (200) und die Keilwelle (222) relativ zu der Verstrebungswelle (232) axial zu bewegen.

7. Rückholvorrichtung nach Anspruch 1,
wobei, wenn der Katheter (200) in einer am meisten proximalen Position ist, die Verstrebungen (230) in einer erweiterten Position sind, und wenn der Katheter (200) in einer am meisten distalen Position ist, die Verstrebungen (230) in einer zusammengelegten Position sind und im Wesentlichen von dem Katheter (200) umgeben sind.

8. Rückholvorrichtung nach Anspruch 1,
wobei der Katheter (200) ferner ein Führungsdrahtlumen (204) umfasst.

9. Rückholvorrichtung nach Anspruch 1,
wobei die Keilwelle (222) einen hohlen Abschnitt aufweist.

10. Rückholvorrichtung nach Anspruch 9,
wobei der hohle Abschnitt der Keilwelle (222) dazu konfiguriert ist, einen Führungsdraht (202) aufzunehmen.

11. Rückholvorrichtung nach Anspruch 1,
wobei der zusammenlegbare Beutel (240) eine Folie oder ein Netz ist.

12. Rückholvorrichtung nach Anspruch 1,
wobei, wenn der Korb (246) aus dem distalen Ende des Katheters (200) austritt, eine axiale Bewegung des Katheters (200) in eine proximale Richtung relativ zu der Verstrebungswelle (232) veranlasst, dass die Vielzahl von Verstrebungen (230) sich auseinander spreizt, bis der zusammenlegbare Beutel gespannt ist.

## Revendications

1. Dispositif de récupération comprenant :
un cathéter (200) ayant au moins une lumière de cathéter, une extrémité proximale et une extrémité distale ;
une tige de renfort (232) ayant une extrémité proximale et une extrémité distale, dans lequel le cathéter (200) entoure sensiblement la tige de renfort (232), et dans lequel le cathéter (200) peut se déplacer axialement par rapport à la tige de renfort (232) ;
un panier (246), dans lequel le panier (246) est configuré pour pouvoir être inséré axialement dans la lumière de cathéter, et comprenant :
une pluralité d'éléments de renfort (230), chacun ayant une extrémité proximale et une extrémité distale, dans lequel l'extrémité proximale de chaque élément de renfort (230) est fixée à l'extrémité distale de la tige de renfort (232) et les extrémités distales des éléments de renfort (230) forment un cadre ; et
une poche pliable (240), dans lequel la poche pliable (240) est reliée de manière flexible à l'extrémité distale de chaque élément de renfort (230) ; et
une partie de commande de dispositif ayant une extrémité proximale et une extrémité distale, dans lequel l'extrémité distale est reliée à l'extrémité proximale du cathéter (200), et le dispositif de récupération comprend en outre :
une tige en coin (222) ayant une extrémité proximale et une extrémité distale ;
un coin (220) ; et
un coulisseau (110) en coin couplé à la tige en coin (222),
dans lequel :
le coin (220) est fixé à l'extrémité distale de la tige en coin (222) ; et la tige de renfort (232) entoure sensiblement la tige en coin (222),
dans lequel
le coin (220) situé à l'extrémité distale de la tige en coin (222) est configuré pour être bloqué contre l'extrémité proximale des éléments de renfort (230) et les éléments de renfort (230) sont écartés, par un mouvement axial dans une direction proximale du coulisseau (110) en coin et de la tige en coin (222).

2. Dispositif de récupération selon la revendication 1,
dans lequel la partie de commande du dispositif est une poignée (100).

3. Dispositif de récupération selon la revendication 2,
dans lequel la poignée (100) comprend en outre :
une pièce de poignée fixe (102) ayant une extrémité proximale et une extrémité distale, et ayant un ou plusieurs anneaux pour les doigts (106, 108) ; et
une pièce de poignée mobile (104) ayant un ou plusieurs anneaux pour les doigts (106, 108),
dans lequel :
la pièce de poignée mobile (104) est configurée pour se déplacer axialement par rapport à la pièce de poignée fixe (102).

4. Dispositif de récupération selon la revendication 3,
dans lequel l'extrémité proximale de la tige de renfort (232) est couplée à l'extrémité distale de la pièce de poignée fixe (102).

5. Dispositif de récupération selon la revendication 3,
dans lequel l'extrémité proximale du cathéter (200) est couplée à la pièce de poignée mobile, et dans lequel la pièce de poignée mobile (104) est configurée pour déplacer le cathéter (200) axialement par rapport à la tige de renfort (232).

6. Dispositif de récupération selon la revendication 3,
dans lequel l'extrémité proximale du cathéter (200) et l'extrémité proximale de la tige en coin (222) sont couplées à la pièce de poignée mobile (104), et dans lequel la pièce de poignée mobile (104) est configurée pour déplacer le cathéter (200) et la tige en coin (222) axialement par rapport à la tige de renfort (232).

7. Dispositif de récupération selon la revendication 1,
dans lequel, lorsque le cathéter (200) est dans une position la plus proximale, les éléments de renfort (230) sont dans une position déployée, et lorsque le cathéter (200) est dans une position la plus distale, les éléments de renfort (230) sont dans une position pliée et sont sensiblement entourées par le cathéter (200).

8. Dispositif de récupération selon la revendication 1,
dans lequel le cathéter (200) comprend en outre une lumière de guide-fil (204).

9. Dispositif de récupération selon la revendication 1,
dans lequel la tige en coin (222) a une partie creuse.

10. Dispositif de récupération selon la revendication 9,
dans lequel la partie creuse de la tige en coin (222) est configurée pour recevoir un fil-guide (202).

11. Dispositif de récupération selon la revendication 1,
dans lequel la poche pliable (240) est un film ou une maille.

12. Dispositif de récupération selon la revendication 1,
dans lequel lorsque le panier (246) sort de l'extrémité distale du cathéter (200), le mouvement axial du cathéter (200) dans une direction proximale par rapport à la tige de renfort (232) provoque l'écartement de la pluralité d'éléments de renfort (230) jusqu'à ce que la poche pliable soit tendue.
